# EUROPEAN PATENT APPLICATION

(11) **EP 2 818 549 A1**
(43) Date of publication of application: **31.12.2014**
(21) Application number: 13752020.1
(22) Date of filing: 22.02.2013
(51) Int. Cl.: C12N 15/09, C12M 1/00, C12Q 1/68, G01N 33/576, G01N 37/00

(54) **METHOD FOR CLASSIFYING TEST BODY FLUID SAMPLE**

(30) Priority: 23.02.2012 JP 2012037586
(71) Applicant: Sumitomo Bakelite Co., Ltd., Shinagawa-ku Tokyo 140-0002 (JP)
(72) Inventor: MURAKAMI Yoshiki, Kyoto-shi Kyoto 615-8084 (JP); OCHIYA Takahiro, Tokyo 104-0045 (JP)
(74) Representative: Albutt, Jodie
(86) International application number: PCT/JP2013/054571
(87) International publication number: WO 2013/125691

(57) **Abstract**

A method for classifying test body fluid samples into either of two groups selected from four groups consisting of chronic hepatitis C, chronic hepatitis B, non-alcoholic steatohepatitis (NASH) and normal liver, is provided. In one or plural embodiment(s), it relates to a method for classifying test body fluid samples into either of two groups selected from four groups consisting of chronic hepatitis C, chronic hepatitis B, non-alcoholic steatohepatitis (NASH) and normal liver. The method includes a measurement of an expression level of the one or plural non-coding RNA(s) in the test body fluid sample.

## Description

### Technical Field

The present invention relates to a method for classifying test body fluid samples into either of two groups selected from four groups consisting of chronic hepatitis B, chronic hepatitis C, non-alcoholic steatohepatitis (NASH) and normal liver; a kit or a microarray used for the method or a use thereof; and a data set to be used for the method.

### Background Art

A mircoRNA has a close relationship with morbidity of chronic liver disorders ranging from chronic hepatitis to hepatoma (Patent document 1, Non-patent documents 1 and 2). Furthermore, a method for diagnosing and detecting cancers by using as a biomarker a mircoRNA derived from exosome is also disclosed (Patent document 2).

### Prior Art Documents

### Patent documents

Patent document 1: JP 2011-217617
Patent document 2: JP 2010-534480

### Non-patent documents

Non-patent document 1: Murakami Y et al. PLoS ONE vol.6 e16081 (2011)
Non-patent document 2: Murakami Y et al. BMC Medical Genomics 3:48 (2010)

### Disclosure of Invention

### Problem to be Solved by the Invention

The present application provides, in one or plural embodiment(s), a method for classifying test body fluid samples into either of two groups selected from four groups consisting of chronic hepatitis C, chronic hepatitis B, non-alcoholic steatohepatitis (NASH) and normal liver.

### Means for Solving Problem

The present application provides, in one or plural embodiment(s), a method for classifying peripheral blood samples into either of two groups selected from four groups consisting of chronic hepatitis C, chronic hepatitis B, non-alcoholic steatohepatitis (NASH) and normal liver. It includes a measurement of an expression level of the one or plural non-coding RNA(s) in the test body fluid samples.

### Effects of the Invention

In one or plural embodiment(s), the present application is enable to classify test body fluid samples into either of two groups selected from four groups consisting of chronic hepatitis C, chronic hepatitis B, non-alcoholic steatohepatitis (NASH) and normal liver.

### Description of the Invention

The present application relates to the following aspects, namely:
[1] a method for classifying test body fluid samples into either of two groups selected from four groups consisting of chronic hepatitis C, chronic hepatitis B, non-alcoholic steatohepatitis (NASH) and normal liver, the method comprising a measurement of an expression level of one or plural non-coding RNA(s) in the test body fluid samples;
[2] the method according to the above [1], wherein the one or plural non-coding RNA(s) comprises hsa-miR-451a;
[3] the method according to the above [1] or [2], further comprising discriminating to which of the two groups the test body fluid samples belong, by use of the expression level of the one or plural non-coding RNA(s) in the test body fluid samples and sample data information obtained from standard body fluid samples of the two groups;
[4] a method for classifying a test body fluid sample as either non-alcoholic steatohepatitis (NASH) or normal liver,
   wherein the method comprises a measurement of an expression level of one or plural non-coding RNA(s) in the test body fluid sample, and the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-1271-5p, hsa-miR-22-3p, hsa-miR-1, hsa-miR-16-5p, and a combination thereof;
[5] a method for classifying a test body fluid sample as either non-alcoholic steatohepatitis (NASH) or chronic hepatitis C,
   wherein the method comprises a measurement of an expression level of one or plural non-coding RNA(s) in the test body fluid sample, and
   the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-483-5p, hsa-miR-320b, hsa-miR-1268a, and a combination thereof;
[6] a method for classifying a test body fluid sample as either non-alcoholic steatohepatitis (NASH) or chronic hepatitis B,
   wherein the method comprises a measurement of an expression level of one or plural non-coding RNA(s) in the test body fluid sample, and
   the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-720, and a combination thereof;
[7] a method for classifying a test body fluid sample as either chronic hepatitis C or normal liver,
   wherein the method comprises a measurement of an expression level of one or plural non-coding RNA(s) in the test body fluid sample, and
   the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-1225-5p, hsa-miR-1275, hsa-miR-1207-5p, and a combination thereof
[8] a method for classifying a test body fluid sample as either chronic hepatitis B or normal liver,
   wherein the method comprises a measurement of an expression level of one or plural non-coding RNA(s) in the test body fluid sample, and
   the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1271-5p, hsa-miR-22-3p, and a combination thereof;
[9] a method for classifying a test body fluid sample as either chronic hepatitis B or chronic hepatitis C,
   wherein the method comprises a measurement of an expression level of one or plural non-coding RNA(s) in the test body fluid sample, and
   the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-483-5p, hsa-miR-320b, hsa-miR-1268a, and a combination thereof;
[10] the method according to any one of the above [1] to [3], wherein the non-coding RNA(s) is a mircoRNA;
[11] a method for classifying a test body fluid sample as either non-alcoholic steatohepatitis (NASH) or normal liver,
   wherein the method comprises a measurement of an expression level of one or plural mircoRNA(s) in the test body fluid sample, and the one or plural mircoRNA(s) is selected from the group consisting of: hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-1, hsa-miR-16, and a combination thereof;
[12] a method for classifying a test body fluid sample as either non-alcoholic steatohepatitis (NASH) or chronic hepatitis C,
   wherein the method comprises a measurement of an expression level of one or plural mircoRNA(s) in the test body fluid sample, and
   the one or plural mircoRNA(s) is selected from the group consisting of: hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-483-5p, hsa-miR-320b, hsa-miR-1268, and a combination thereof
[13] a method for classifying a test body fluid sample as either non-alcoholic steatohepatitis (NASH) or chronic hepatitis B,
   wherein the method comprises a measurement of an expression level of a mircoRNA in the test body fluid sample, and
   the mircoRNA is hsa-miR-451a;
[14] a method for classifying a test body fluid sample as either chronic hepatitis C or normal liver,
   wherein the method comprises a measurement of an expression level of one or plural mircoRNA(s) in the test body fluid sample, and
   the one or plural mircoRNA(s) is selected from the group consisting of: hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-483-5p, and a combination thereof
[15] a method for classifying a test body fluid sample as either chronic hepatitis B or normal liver,
   wherein the method comprises a measurement of an expression level of one or plural mircoRNA(s) in the test body fluid sample, and
   the one or plural mircoRNA(s) is selected from the group consisting of: hsa-miR-451a, hsa-miR-638, hsa-miR-762, hsa-miR-320c, hsa-miR-92a, hsa-miR-486-5p, hsa-miR-630, hsa-miR-1246, and a combination thereof;
[16] a method for classifying a test body fluid sample as either chronic hepatitis B or chronic hepatitis C,
   wherein the method comprises a measurement of an expression level of one or plural mircoRNA(s) in the test body fluid sample, and
   the one or plural mircoRNA(s) is selected from the group consisting of hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-483-5p, hsa-miR-320b, hsa-miR-1268, and a combination thereof
[17] the method according to any one of the above [1] to [16], wherein the body fluid sample is a blood sample;
[18] the method according to any one of the above [1] to [17], wherein the non-coding RNA or the mircoRNA is a RNA in an exosome-rich fraction of the body fluid sample;
[19] a method for classifying test body fluid samples into any of four groups consisting of chronic hepatitis C, chronic hepatitis B, non-alcoholic steatohepatitis (NASH) and normal liver, the method comprising a classification of the peripheral blood samples by using the method according to any one of the above [1] to [18];
[20] use of a non-coding RNA selected from the group consisting of hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-1271-5p, hsa-miR-22-3p, hsa-miR-483-5p, hsa-miR-320b, hsa-miR-1268a, hsa-miR-1, hsa-miR-16-5p, and a combination thereof, as a marker of chronic hepatitis C, chronic hepatitis B, and/or non-alcoholic steatohepatitis (NASH);
[21] use of a mircoRNA selected from the group consisting of hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-483-5p, hsa-miR-320b, hsa-miR-1268, hsa-miR-1, hsa-miR-16, and a combination thereof, as a marker of chronic hepatitis C, chronic hepatitis B, and/or non-alcoholic steatohepatitis (NASH);
[22] a realtime PCR kit used for the method according to any one of above [1] to [19], comprising a primer and a reagent capable of measuring an expression level of one or plural non-coding RNA(s) in the test body fluid sample;
[23] a microarray used for the method according to any one of the above [1] to [19], comprising a probe capable of measuring an expression level of one or plural non-coding RNA(s) in the test body fluid sample;
[24] a method of using the kit according to the above [22] or the microarray according to the above [23], comprising a measurement of the one or plural non-coding RNA(s) in the method according to any one of the above [1] to [19], using the kit or the microarray;
[25] a data set comprising expression data of one or plural non-coding RNA(s) selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-1271-5p, hsa-miR-22-3p, hsa-miR-483-5p, hsa-miR-320b, hsa-miR-1268a, hsa-miR-1, hsa-miR-16-5p, and a combination thereof in the body fluid sample,
   the data set comprises expression data of said non-coding RNA obtained from a standard body fluid sample of an individual belonging to at least one group selected from four groups consisting of chronic hepatitis C, chronic hepatitis B, non-alcoholic steatohepatitis (NASH) and normal liver, and the data set is to be used in the method according to any one of the above [1] to [19];
[26] use of a next-generation sequencer in the method according to any one of the above [1] to [19], comprising a measurement of an expression level of one or plural non-coding RNA(s) in the test body fluid sample by use of the next-generation sequencer; and
[27] a kit for performing the method according to any one of the above [1] to [19] with a next-generation sequencer, the kit comprising a primer and a reagent capable of measuring an expression level of the one or plural non-coding RNA(s) in the test body fluid sample.

In one or plural embodiment(s), "non-coding RNA" includes mircoRNA and tRNA. In one or plural embodiment(s), "mircoRNA/miRNA" is a type of low-molecular non-coding RNA, and the term refers to a normal meaning used in the technical field. In the present specification, the data base can be conferred with respect to the mircoRNA indicated by use of ID. In the present specification, except where specifically noted, data of Release 18 (November, 2011) of miRBase (http://www.miRbase.org/) is conferred with respect to the titles and accession numbers of the mircoRNA. And in the present specification, except where specifically noted, the mircoRNA indicates a mature mircoRNA composed of 16-25 bases.

In the present specification, "body fluid" refers to a body fluid including exosome, and in one or plural embodiment(s), it may include blood, lymph, ascites, pleural fluid, saliva, urine, and breast milk. From the viewpoint of convenience in collection and reduction of stress imposed on a subject, the blood is preferably a peripheral blood. In one or plural embodiment(s), "body fluid sample" includes the body fluid or any sample obtainable from the body fluid. In one or plural embodiment(s), examples of the body fluid sample include blood plasma and serum. Furthermore in one or plural embodiment(s), the body fluid sample may be a fraction formed by concentrating or roughly-refining exosome in the body fluid (hereinafter, referred to also as "exosome-rich fraction") or exosome formed by isolating or refining the body fluid. From the viewpoint of reproducibility in detection of the expression level of either the non-coding RNA or the mircoRNA, it is preferable in one or plural embodiment(s) that the body fluid sample is either the exosome-rich fraction or the exosome formed by isolating or refining. Preparation of exosome-rich fraction and/or isolation or refining of exosome can be performed in accordance with any known method or by using a commercially-available kit etc. Further, the method can be performed in accordance with the recitation of Examples mentioned below. Therefore, in one or plural embodiment(s), from the viewpoint of convenience in collection and reduction of stress imposed on a subject, and also from the viewpoint of reproducibility of detection of the expression level of either the non-coding RNA or the mircoRNA, it is preferable that the body fluid sample is exosome-rich fraction prepared from blood plasma or serum of peripheral blood.

In this specification, "test body fluid sample" indicates a body fluid sample to be classified by a classification method. In the present specification, "standard body fluid sample" indicates a body fluid sample obtained from an individual known as belonging to any one of chronic hepatitis C, chronic hepatitis B, non-alcoholic steatohepatitis (NASH) and normal liver. In the present specification, a "body fluid sample" may include both the above-described test body fluid sample and the standard body fluid sample, unless otherwise specified.

In the present specification, "exosome" is a kind of membrane vesicle secreted from the cells, generally having a diameter in the range of 30 to 100 nm in an observation by a scanning electron microscope, and having a density distributed in a fraction of about 1.13 g/ml due to a density gradient ultracentrifugation. A cell envelops unnecessary proteins and amino acids in the cytoplasm with membrane structure (exosome) and transports them outside the cell. It is known that the exosome circulates in the body fluid. Furthermore, it is reported recently that exosome relates to intercellular information exchange. It has been clarified also that a cancer cell secretes its own mircoRNA sealed in exosome (see the above Patent document 2 for example).

An example of the individual as the source of the body fluid is a human being or an animal as a non-human being. In one or plural embodiment(s), an example of the non-human being is a mammal, and in particular, primate, rodentia and the like.

In the present specification, "chronic hepatitis C" indicates a disorder where in general an infection to hepatitis C virus (HCV) occurs and abnormality in liver function (hepatitis) continues (generally for 6 months or more). Hereinafter, the chronic hepatitis C may be expressed also as "CHC". In the present specification, "chronic hepatitis B" indicates a disorder where in general an infection to hepatitis B virus (HBV) occurs and abnormality in liver function (hepatitis) continues (generally for 6 months or more). Hereinafter, the chronic hepatitis B may be expressed also as "CHB". It is said that in many cases of such chronic hepatitis, the disease develops slowly to hepatocirrhosis, which further transforms to hepatoma. The chronic hepatitis C and the chronic hepatitis B are diagnosed by medical doctors in general with reference to information such as detection of infection of hepatitis C/B virus and hepatitis (abnormality in liver function) based on blood examination, ultrasonic inspection, and CT/MRI, for example. Furthermore, for a more detailed examination of the disorders and for checking the advancement, a liver biopsy may be performed. In general, detection of hepatitis or an abnormality in liver function by blood examination is performed by measurement of enzyme level (e.g., IU/L unit) of AST ((aspartate aminotransferase), which is called as also GOT (glutamic oxaloacetic transaminase)) and ALT ((alanine aminotransferase), which is called also as GPT (glutamate pyruvate transferase)), but the present invention is not limited to these examples.

In the present specification, "non-alcoholic steatohepatitis (NASH)" indicates a disorder where storage of lipid including mainly neutral lipid occurs in the liver cells so that pathological findings similar to those of alcoholic liver injury are observed even for a nondrinker (for example, less than 20 g per day in terms of ethanol). Conventionally, it has been considered that fatty liver (hepar adiposum) is a benign reversible disease. However, it has been found that a part of fatty liver such as NASH may progress to hepatocirrhosis and to hepatoma, and thus, the seriousness as one adult disease is recognized. The diagnosis of NASH is to satisfy the following three requirements; (1) nondrinker (for example, less than 20 g per day in terms of ethanol); (2) observation result on the liver structure is fatty liver (deposition of big drop fat, inflammatory cell infiltrating, ballooning hepatic cell, and the like); and (3) exclusion of liver disorders caused by other factors.

Namely at present, liver biopsy in diagnosis of NASH is regarded as the best way. However, liver biopsy is regarded as imposing stress on the patient (i.e., invasive). And thus, if any clinical information can be obtained while avoiding the liver biopsy, the stress of patient can be reduced. In one or plural embodiment(s), the present application is based on a finding that, on the basis of the expression level or expression pattern of one or plural non-coding RNA(s) in the test body fluid samples, the test body fluid samples can be classified into either of two groups selected group four groups consisting of chronic hepatitis C, chronic hepatitis B, non-alcoholic steatohepatitis (NASH) and normal liver.

In the present specification, "normal liver" indicates a liver that is not diagnosed as chronic hepatitis C, chronic hepatitis B, or non-alcoholic steatohepatitis (NASH). In one or plural embodiment(s), it indicates a liver that either hepatitis or any liver function abnormality is not detected in a blood examination (for example, measurement of AST or ALT). Hereinafter, normal liver may be recited also as "NL".

### [Classification method]

In one or plural embodiment(s), the present application relates to a method of classifying test body fluid samples into either of two groups that can be selected from four groups consisting of chronic hepatitis C, chronic hepatitis B, non-alcoholic steatohepatitis (NASH) and normal liver (hereinafter, this is referred to also as "classification method of the present application"). In one aspect, the classification method of the present application is an objective or statistical classification method, which does not correspond to or include a diagnosis. The result according to the classification method of the present application may make data for supporting diagnoses.

A classification method in First Embodiment is a method for classifying a test body fluid sample as either non-alcoholic steatohepatitis (NASH) or normal liver. A classification method in Second Embodiment is a method for classifying a test body fluid sample as either non-alcoholic steatohepatitis (NASH) or chronic hepatitis C. A classification method in Third Embodiment is a method for classifying a test body fluid sample as either non-alcoholic steatohepatitis (NASH) or chronic hepatitis B. A classification method in Fourth Embodiment is a method for classifying a test body fluid sample as either chronic hepatitis C or normal liver. A classification method in Fifth Embodiment is a method for classifying a test body fluid sample as either chronic hepatitis B or normal liver. And a classification method in Sixth Embodiment is a method for classifying a test body fluid sample as either chronic hepatitis B or chronic hepatitis C. The classification methods in First to Sixth Embodiments may or may not include "a step of selecting two groups from four groups consisting of chronic hepatitis C, chronic hepatitis B, non-alcoholic steatohepatitis (NASH) and normal liver". A classification method in Seventh Embodiment is a method for classifying the test body fluid sample from all or a part of the results according to the classification methods of First to Sixth Embodiments as chronic hepatitis C, chronic hepatitis B, non-alcoholic steatohepatitis (NASH) or normal liver.

The classification method of the present application includes an measurement of an expression level of one or plural non-coding RNA(s) in the test body fluid sample. In the present specification, "measurement of expression level of non-coding RNA in the body fluid sample" indicates calculation of RNA amount of a predetermined sequence included in the body fluid sample. In one or plural embodiment(s), the amount of RNA may be an absolute value or a value relative to the internal standard. The measurement of the non-coding RNA may be performed by any known methods or any methods to be developed in the future, and a commercially-available kit can be utilized suitably. In a specific and non-limitative example, cDNA is synthesized from RNA in the body fluid sample, which is then measured by a quantitative PCR, a realtime quantitative PCR, a microarray or the like. In the present specification, "non-coding RNA used for classification method" indicates one or plural non-coding RNA(s) of a target for a measurement of an expression level, and indicates a measurement of the non-coding RNA(s) in the classification method.

In one or plural embodiment(s), the classification method of the present application further includes discriminating to which of two groups the test body fluid sample belongs, by use of the expression level of the one or plural non-coding RNA(s) in the test body fluid sample and the sample data information obtained from the standard body fluid samples of the two groups.

As mentioned above, a standard body fluid sample indicates a body fluid sample obtained from an individual that is known as belonging to any one group of chronic hepatitis C, chronic hepatitis B, non-alcoholic steatohepatitis (NASH) and normal liver. The determination whether the individual belongs to any of the groups can depend on the diagnosis by the doctor. In one or plural embodiment(s), it can be decided that an individual that belongs to plural groups, or an individual that corresponds to any of superinfection of HIV-1 or HIV-2, decompensated liver cirrhosis, organ transplant, immunosuppression, autoimmune disorder, alcohol intake over 20 g per day, or anamnesis of intravenous drug abuse is excluded from individuals belonging to the respective groups.

In one or plural embodiment(s), "sample data information" in the present specification indicates data of an expression level of one or plural non-coding RNA(s) in the standard body fluid samples of the group to be classified and/or information obtained from said data. The number of standard body fluid samples that can be included in the sample data information is at least one per group. The number is preferably at least 2 from the viewpoint of improving accuracy in classification, more preferably at least 4, and further preferably at least 7, namely, a larger number is preferred. There is no particular upper limit of the number of standard body fluid samples as long as calculation for discrimination is available. In one or plural embodiment(s), the one or plural non-coding RNA(s) of the test body fluid sample and one or plural non-coding RNA(s) of the standard body fluid sample are common.

In one or plural embodiment(s), the discrimination can be performed by a discriminant analysis. In one or plural embodiment(s), the discriminant analysis may be a discrimination using a discriminant function or a discrimination based on the distance. In one or plural embodiment(s), the discriminant function may be a linear function or nonlinear function. In one or plural embodiment(s), the discrimination by the distance can use Mahalanobis distance. In one or plural embodiment(s), the discriminant analysis can be performed by using statistical analysis software such as R. Therefore in one or plural embodiment(s), the "sample data information" may include a discriminant function or a discriminant threshold for discrimination, in addition to or in place of data such as the kind of non-coding RNA that brings about an expression level to make a variation of the discriminant analysis and the expression level.

From the viewpoint of improving accuracy of the classification, it is preferable that the method of measuring the expression level of the non-coding RNA in the test body fluid sample and the method of measuring the expression level of the non-coding RNA used or included in the sample data information are common.

### [Non-coding RNA used for classification]

In one or plural embodiment(s), the classification method of the present application includes at least hsa-miR-451a as the non-coding RNA to be used for classification. And in one or plural embodiment(s), in the classification method of the present application, it is possible to make all or a part of the non-coding RNAs indicated in Table 1 below as the non-coding RNA to be used for classification. The non-coding RNAs indicated in Table 1 below are mircoRNAs registered in the data of Release 18 (November 2011) of miRBase (http://www.miRbase.org/) except for has-miR-1974. Meanwhile, has-miR-1974 is regarded currently as mitochondria tRNA. Therefore, in one or plural embodiment(s), one example of the classification method of the present application is "an embodiment using non-coding RNA" including has-miR-1974, while in another or the other embodiment(s), the example is "an embodiment using mircoRNA" that does not use has-miR-1974.

### [Table 1]

**(Table 1)**

| miRNA | Accession No. | Sequence (5'→3') | Seq. ID |
|---|---|---|---|
| hsa-miR-638 | MIMAT0003308 | AGGGAUCGCGGGCGGGUGGCGGCCU | 1 |
| hsa-miR-762 | MIMAT0010313 | GGGGCUGGGGCCGGGGCCGAGC | 2 |
| hsa-miR-320c | MIMAT0005793 | AAAAGCUGGGUUGAGAGGGU | 3 |
| hsa-miR-451a | MIMAT0001631 | AAACCGUUACCAUUACUGAGUU | 4 |
| hsa-miR-1974 | (miRBase ver 14.0) | AGUCCGUGCGAGAAUA | 5 |
| hsa-miR-1246 | MIMAT0005898 | AAUGGAUUUUUGGAGCAGG | 6 |
| hsa-miR-92a-3p | MIMAT0000092 | UAUUGCACUUGUCCCGGCCUGU | 7 |
| hsa-miR-486-5p | MIMAT0002177 | UCCUGUACUGAGCUGCCCCGAG | 8 |
| hsa-miR-630 | MIMAT0003299 | AGUAUUCUGUACCAGGGAAGGU | 9 |
| hsa-miR-1225-5p | MIMAT0005572 | GUGGGUACGGCCCAGUGGGGGG | 10 |
| hsa-miR-1275 | MIMAT0005929 | GUGGGGGAGAGGCUGUC | 11 |
| hsa-miR-720 | MIMAT0005954 | UCUCGCUGGGGCCUCCA | 12 |
| hsa-miR-1207-5p | MIMAT0005871 | UGGCAGGGAGGCUGGGAGGGG | 13 |
| hsa-miR-1202 | MIMAT0005865 | GUGCCAGCUGCAGUGGGGGAG | 14 |
| hsa-miR-1915-3p | MIMAT0007892 | CCCCAGGGCGACGCGGCGGG | 15 |
| hsa-miR-320d | MIMAT0006764 | AAAAGCUGGGUUGAGAGGA | 16 |
| hsa-miR-1271-5p | MIMAT0005796 | CUUGGCACCUAGCAAGCACUCA | 17 |
| hsa-miR-22-3p | MIMAT0000077 | AAGCUGCCAGUUGAAGAACUGU | 18 |
| hsa-miR-483-5p | MIMAT0004761 | AAGACGGGAGGAAAGAAGGGAG | 19 |
| hsa-miR-320b | MIMAT0005792 | AAAAGCUGGGUUGAGAGGGCAA | 20 |
| hsa-miR-1268a | MIMAT0005922 | CGGGCGUGGUGGUGGGGG | 21 |
| hsa-miR-1 | MIMAT0000416 | UGGAAUGUAAAGAAGUAUGUAU | 22 |
| hsa-miR-16-5p | MIMAT0000069 | UAGCAGCACGUAAAUAUUGGCG | 23 |

As an embodiment to use a non-coding RNA in a classification method of First Embodiment for classification as non-alcoholic steatohepatitis (NASH) or normal liver, a non-coding RNA selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-1271-5p, hsa-miR-22-3p, hsa-miR-1, hsa-miR-16-5p, and a combination thereof is used. From the viewpoint of improving accuracy in the classification, it is preferable to use two or more of the above-described twenty non-coding RNAs. It is more preferable to use a combination of five, i.e., hsa-miR-638, hsa-miR-762, hsa-miR-451a, hsa-miR-1974 and hsa-miR-1246 or all of the twenty RNAs (see Table 3 below). In the specification, the combination in "x₁, x₂, ... xₙ, and a combination thereof" includes a combination of numbers of 2 to n elements selected from the group consisting of x₁, x₂, ... and xₙ.

As an embodiment to use a mircoRNA in the classification method of First Embodiment, a mircoRNA selected from the group consisting of: hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-1, hsa-miR-16, and a combination thereof is used. From the viewpoint of improving accuracy in the classification, it is preferable to use two or more of the above-described eighteen mircoRNAs, and it is more preferable to use all of them (see Table 6 below).

As an embodiment to use a non-coding RNA in a classification method of Second Embodiment for classification as non-alcoholic steatohepatitis (NASH) or chronic hepatitis C, a non-coding RNA selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-483-5p, hsa-miR-320b, hsa-miR-1268a, and a combination thereof is used. From the viewpoint of improving accuracy in the classification, it is preferable to use two or more of the above-described nineteen non-coding RNAs, and it is more preferable to use all of them (see Table 3 below).

As an embodiment to use a mircoRNA in the classification method of Second Embodiment, a mircoRNA selected from the group consisting of: hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-483-5p, hsa-miR-320b, hsa-miR-1268, and a combination thereof is used. From the viewpoint of improving accuracy in the classification, it is preferable to use two or more of the above-described nineteen microRNAs, and it is more preferable to use all of them (see Table 6 below).

As an embodiment to use a non-coding RNA in a classification method of Third Embodiment for classification as non-alcoholic steatohepatitis (NASH) or chronic hepatitis B, a non-coding RNA selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-720, and a combination thereof is used. From the viewpoint of improving accuracy in the classification, it is preferable to use two or more of the above-described ten non-coding RNAs, and it is more preferable to use all of them (see Table 3 below).

As an embodiment to use a mircoRNA in the classification method of Third Embodiment, hsa-miR-451a is used (see Table 6 below).

As an embodiment to use a non-coding RNA in a classification method of Fourth Embodiment for classification as chronic hepatitis C or normal liver, a non-coding RNA selected from the group consisting of hsa-miR-638, hsa-miR-762, hsa-miR-320c, hsa-miR-451a, hsa-miR-1974, hsa-miR-1246, hsa-miR-1225-5p, hsa-miR-1275, hsa-miR-1207-5p, and a combination thereof is used. From the viewpoint of improving accuracy in the classification, it is preferable to use two or more of the above-described nine non-coding RNAs, and it is more preferable to use all of them (see Table 3 below).

As an embodiment to use a mircoRNA in the classification method of Fourth Embodiment, a mircoRNA selected from the group consisting of hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-483-5p, and a combination thereof is used. From the viewpoint of improving accuracy in the classification, it is preferable to use two or more of the above-described fifteen mircoRNAs, and it is more preferable to use all of them (see Table 6 below).

As an embodiment to use a non-coding RNA in a classification method of Fifth Embodiment for classification as chronic hepatitis B or normal liver, a non-coding RNA selected from the group consisting of hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1271-5p, hsa-miR-22-3p, and a combination thereof is used. From the viewpoint of improving accuracy in the classification, it is preferable to use two or more of the above-described eleven non-coding RNAs, and it is more preferable to use all of them (see Table 3 below).

As an embodiment to use a mircoRNA in the classification method of Fifth Embodiment, a mircoRNA selected from the group consisting of hsa-miR-451a, hsa-miR-638, hsa-miR-762, hsa-miR-320c, hsa-miR-92a, hsa-miR-486-5p, hsa-miR-630, hsa-miR-1246, and a combination thereof is used. From the viewpoint of improving accuracy in the classification, it is preferable to use two or more of the above-described eight mircoRNAs, and it is more preferable to use all of them (see Table 6 below).

As an embodiment to use a non-coding RNA in a classification method of Sixth Embodiment for classification as chronic hepatitis B or chronic hepatitis C, a non-coding RNA selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-483-5p, hsa-miR-320b, hsa-miR-1268a, and a combination thereof is used. From the viewpoint of improving accuracy in the classification, it is preferable to use two or more of the above-described nineteen non-coding RNAs, and it is more preferable to use all of them (see Table 3 below).

As an embodiment to use a mircoRNA in the classification method of Sixth Embodiment, a mircoRNA selected from the group consisting of: hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-483-5p, hsa-miR-320b, hsa-miR-1268, and a combination thereof is used. From the viewpoint of improving accuracy in the classification, it is preferable to use two or more of the above-described nineteen mircoRNAs, and it is more preferable to use all of them (see Table 6 below).

In the classification methods in one or more of the above-described First to Sixth Embodiments, a mircoRNA except the ones in the above Table 1 may be used.

As mentioned above, the classification method in Seventh Embodiment is a method of classifying the test body fluid sample from all or a part of the results in the classification methods in the First to the Sixth Embodiments, as any of chronic hepatitis C, chronic hepatitis B, non-alcoholic steatohepatitis (NASH) and normal liver.

### [Marker]

Expression level of the non-coding RNA in the body fluid as shown in Table 1 can be used for classification of the test body fluid samples into chronic hepatitis C, chronic hepatitis B, non-alcoholic steatohepatitis (NASH) and normal liver, as mentioned above. Therefore, in another aspect, the present application relates to a use of a non-coding RNA selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-1271-5p, hsa-miR-22-3p, hsa-miR-483-5p, hsa-miR-320b, hsa-miR-1268a, hsa-miR-1, hsa-miR-16-5p, and a combination thereof, namely a use as a marker of chronic hepatitis C, chronic hepatitis B, and/or non-alcoholic steatohepatitis (NASH). These markers can be used for one or plural embodiment(s) using non-coding RNAs in the above-mentioned classification methods according to First to Seventh Embodiments.

Furthermore, in a further aspect, the present application relates to a use of a mircoRNA selected from the group consisting of: hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-483-5p, hsa-miR-320b, hsa-miR-1268, hsa-miR-1, hsa-miR-16, and a combination thereof, namely a use as a marker of chronic hepatitis C, chronic hepatitis B, and/or non-alcoholic steatohepatitis (NASH). These markers can be used for one or plural embodiment(s) using mircoRNAs in the above-mentioned classification methods according to First to Seventh Embodiments.

### [Kit]

The present application relates to a quantitative PCR kit used for the classification method of the present application in any other aspects, which is a kit including a primer and a reagent for allowing a measurement of the expression level of one or plural non-coding RNA(s) in a test body fluid sample. In one or plural embodiment(s), the one or plural non-coding RNA(s) may be the all or a part of the ones in Table 1 above. In one or plural embodiment(s), the primer can be selected suitably in accordance with the classification methods in the above-mentioned First to Seventh Embodiments. In one or plural embodiment(s), the kit of the present application further relates to a kit including an instruction manual reciting the classification method of the present application. The kit of the present application may include a case where the instruction manual is provided on the web without being packaged with the kit of the present application. In one or plural embodiment(s), the kit of the present application may include a primer and a reagent for synthesizing cDNA. In a further aspect, the present application relates to a use of the kit in the classification method of the present application, and specifically, a use in a measurement of one or plural non-coding RNA(s).

In another aspect, the present application relates to a kit for performing the classification method of the present application with the next-generation sequencer, and the kit includes a primer and a reagent for measuring the expression level of one or plural non-coding RNA(s) in the test body fluid sample. In one or plural embodiment(s), the embodiment relates to a kit for preparing a sample for performing the classification method of the present application with the next-generation sequencer. In one or plural embodiment(s), examples of the one or plural non-coding RNA(s) are the all or a part of the ones recited in Table 1. In one or plural embodiment(s), the primer can be selected suitably in accordance with the classification methods in the above-mentioned First to Seventh Embodiments. In one or plural embodiment(s), the kit of the present application relates to a kit including an instruction manual reciting the classification method of the present application. The kit of the present application may include a case where the instruction manual is provided on the web without being packaged with the kit of the present application. Examples of the reagents in the present application may include an enzyme, a label sequence, and/or a buffer and the like. In a further aspect, the present application relates to a use of the next-generation sequencer in the classification method of the present application, specifically, a use of the next-generation sequencer, including measurement of expression level of one or plural non-coding RNA(s) in the test body fluid sample by use of the next-generation sequencer. In the present application, a next-generation sequencer indicates a sequencer classified in comparison with a fluorescent capillary sequencer utilizing a Sanger sequencing method as "first-generation sequencer", and it includes any sequencers of the second generation, the third generation, the fourth generation and any sequencers to be developed in the future, which do not utilize the Sanger sequencing method.

### [Microarray]

In another aspect, the present application relates to a microarray to he used in the classification method of the present application, namely a microarray including a probe that can measure the expression level of one or plural non-coding RNA(s) in the test body fluid sample. In one or plural embodiment(s), the one or plural non-coding RNA(s) may be the all or a part of the ones recited in Table 1. In one or plural embodiment(s), the probe can be selected suitably in accordance with the classification methods in the above-mentioned First to Seventh Embodiments. In another aspect, the present application relates to a use of the microarray in the classification method of the present application, specifically a use in measurement of one or plural non-coding RNA(s).

### [Data set]

In another aspect, the present application relates to a data set including expression data of one or plural non-coding RNA(s) selected from the group consisting of hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-1271-5p, hsa-miR-22-3p, hsa-miR-483-5p, hsa-miR-320b, hsa-miR-1268a, hsa-miR-1, hsa-miR-16-5p, and a combination thereof in body fluid samples, which is a data set including the expression data of the non-coding RNAs obtained from a standard body fluid sample of an individual belonging to at least one group selected from the groups consisting of chronic hepatitis C, chronic hepatitis B, non-alcoholic steatohepatitis (NASH) and normal liver. In one or plural embodiment(s), the data set of the present application can be used as the sample data information in the classification method of the present application.

### Examples

By using the miRNA expression pattern of peripheral blood, by the following methods and under the conditions, combinations of mircoRNAs that can classify CHC (chronic hepatitis C), CHB (chronic hepatitis B), NASH (non-alcoholic steatohepatitis) and NL (normal liver) were searched.

### 1. Blood (peripheral blood) sample

Peripheral blood samples were obtained from sixty-four CHC patients, four CHB patients, seven NASH patients, and twenty persons having normal livers. The patients do not include persons having any of superinfection of HIV-1 or HIV-2, decompensated liver cirrhosis, organ transplant, immunosuppression, autoimmune disorder, alcohol intake over 20 g per day, or anamnesis of intravenous drug abuse. Further, each patient was subjected to a biopsy of liver in advance. Determination of a NASH patient was conducted in a histological manner. Diagnoses of CHC and CHB were conducted through checkups of virus infection, liver functions and the like. All of the patients or the parents of the patients submitted written forms of informed consent. Furthermore, all of the aspects regarding this study have been admitted by the Institutional Review Boards of Ogaki Municipal Hospital and Graduate School of Medicine and Faculty of Medicine Kyoto University in accordance with the Declaration of Helsinki.

The peripheral blood was collected from each individual directly into a serum tube before conducting an antiviral treatment. The serum tube was subjected to a 1500 g centrifugation for 10 minutes at 4°C. The collected serum moiety was further subjected to a 2000 g centrifugation so as to remove the cells completely, thereby making serum samples. The serum samples were preserved at -80°C.

### 2. Preparation of RNA

Total RNA was prepared from 200 µl serum samples by using a miRNeasy mini kit (manufactured by Qiagen) in accordance with the instruction manual. The exosome-rich fractional RNA was prepared by using Exoquick (manufactured by System Biosciences Inc.). Specifically, 900 µl of serum sample was mixed with 250 µl of Exoquick and then incubated at 4°C for 12 hours. Later, the sample was subjected to a 1500 g centrifugation for 30 minutes, and the supernatant was discarded. The residual pellet was dissolved in a 200 µl PBS, and RNA was prepared by using miRNeasy mini kit (manufactured by Qiagen) in accordance with the instruction manual.

### 3. miRNA microarray

In the detection of miRNA in a serum sample by use of the microarray, Human microRNA Microarray Kit (Rel 14.0) (manufactured by Agilent Technologies) was used. In accordance with the instruction manual (for Agilent microRNA microarrays Version 1.0), 60 ng RNA was labeled and hybridized. The hybridization signal was detected by using DNA microarray scanner G250B (manufactured by Agilent Technologies). The scanned image was analyzed by using Agilent feature extraction software (v9.5.3.1). Raw data (gProcessedSignal) were used and each expression was normalized to have a zero-mean value and a unit sampling distribution. This process was performed by using R.

### 4. Realtime qPCR

Detection of the miRNA in the serum samples by realtime quantitative PCR was performed by using TaqMan (trademark) mircoRNA assay (manufactured by Applied Biosystems Inc.). The expression level of miR-16 was used as the internal control. The cDNA was produced by using TaqMan (trademark) miRNA RT Kit (manufactured by Applied Biosystems Inc.). Detection of expression of the miRNA was performed by use of Chromo 4 detector (manufactured by Bio-Rad Laboratories, Inc.).

### 5. Statistical analysis

For symptoms having discrete values, two combinations were compared in Wilcoxon rank-sum test (one-side), or the P-value was calculated on the basis of the correlation function. In both cases, it was regarded as significant when FDR (false discovery rate) was less than 0.05.

### [Selection of miRNA characteristic to liver disorders]

For a pair of any liver disorders (CHC, CHB, NASH) and normal liver, the expression levels of normalized miRNA were transformed to a main ingredient score by a main ingredient analysis. A miRNA having a larger first main ingredient score and second main ingredient score was selected. Next, the first main ingredient score of each sample was calculated on the basis of the expression level of only the selected miRNA. Based on these, a first main ingredient score of the optimal number was used to classify the liver disorders and normal liver. These operations were conducted by R.

### 6. Results

(1) As to the expression level of miRNA in the serum sample, from the viewpoint of reproducibility, it has been found that a RNA that is collected from an exosome-rich fraction prepared from a serum sample is superior to a total RNA collected from a serum sample (no data).
(2) As to the six pairs selected from the liver disorders (CHC, CHB, NASH) and the normal liver (NL), respectively appropriate miRNAs were selected as mentioned above, thereby discrimination with favorable accuracy was realized as shown in Table 2 below (Table 2-1 to Table 2-6).

### [Table 2]

**(Table 2)**

| Table 2-1 | result | | | |
|---|---|---|---|---|
| prediction | | NASH | NL | |
| | NASH | 6 | 5 | |
| | NL | 1 | 15 | accuracy 77.8% |
| | | | | |

| Table 2-2 | result | | | |
|---|---|---|---|---|
| Prediction | | CHC | NASH | |
| | CHC | 62 | 0 | |
| | NASH | 2 | 7 | accuracy 97.2% |
| | | | | |

| Table 2-3 | result | | | |
|---|---|---|---|---|
| Prediction | | CHB | NASH | |
| | CHB | 3 | 1 | |
| | NASH | 1 | 6 | accuracy 81.8% |
| | | | | |

| Table 2-4 | result | | | |
|---|---|---|---|---|
| prediction | | CHC | NL | |
| | CHC | 64 | 2 | |
| | NL | 0 | 18 | accuracy 97.6% |
| | | | | |

| Table 2-5 | result | | | |
|---|---|---|---|---|
| prediction | | CHB | NL | |
| | CHB | 3 | 6 | |
| | NL | 1 | 14 | accuracy 70.8% |
| | | | | |

| Table 2-6 | result | | | |
|---|---|---|---|---|
| prediction | | CHB | CHC | |
| | CHB | 3 | 0 | |
| | CHC | 1 | 64 | accuracy 98.5% |

Table 3 below shows miRNAs used for discriminating the six pairs in Table 2. The expression levels of miRNAs indicated by "*" in Table 3 were used.

### [Table 3]

**(Table 3)**

| miRNA | NASHvsNL | NASHvsCHC | NASHvsCHB | NLvsCHC | NLvsCHB | CHCvsCHB |
|---|---|---|---|---|---|---|
| hsa-miR-638 | * | * | * | * | * | * |
| hsa-miR-762 | * | * | * | * | * | * |
| hsa-miR-320c | * | * | * | * | * | * |
| hsa-miR-451a | * | * | * | * | * | * |
| hsa-miR-1974 | * | * | * | * | * | * |
| hsa-miR-1246 | * | * | * | * | * | * |
| hsa-miR-92a-3p | * | * | * | | * | * |
| hsa-miR-486-5p | * | * | * | | * | * |
| hsa-miR-630 | * | * | * | | * | * |
| hsa-miR-1225-5p | * | * | | * | | * |
| hsa-miR-1275 | * | * | | * | | * |
| hsa-miR-720 | * | * | * | | | * |
| hsa-miR-1207-5p | * | * | | * | | * |
| hsa-miR-1202 | * | * | | | | * |
| hsa-miR-1915-3p | * | * | | | | * |
| hsa-miR-320d | * | * | | | | * |
| hsa-miR-1271-5p | * | | | | * | |
| hsa-miR-22-3p | * | | | | * | |
| hsa-miR-483-5p | | * | | | | * |
| hsa-miR-320b | | * | | | | * |
| hsa-miR-1268a | | * | | | | * |
| hsa-miR-1 | * | | | | | |
| hsa-miR-16-5p | * | | | | | |

Table 4 below shows the result of a calculation performed by organizing the results in Table 2 into a 4x4 contingency table for calculating the accuracy.

### [Table 4]

**(Table 4)**

| | Result | | | | | |
|---|---|---|---|---|---|---|
| prediction | | CHC | NL | CHB | NASH | |
| | CHC | 64 | 2 | 1 | 1 | |
| | NL | 0 | 17 | 0 | 1 | |
| | CHB | 0 | 1 | 3 | 1 | accuracy 92.6% |
| | NASH | 0 | 0 | 0 | 4 | |

As shown in Table 2 to Table 4, it has been found that classification of liver disorders (CHC, CHB, NASH) and normal liver can be performed based on the expression level of miRNA in the blood samples shown in Table 2.

### 7. Aspect not using has-miR-1974

For the miRNA in Table 3, date of Release 18 of miRBase (www.mirbase.org) are referred to except for has-miR-1974. Meanwhile, has-miR-1974 is dead entry in Release 18 data and regarded as a mitochondria tRNA. Therefore, without using has-miR-1974, regarding the six pairs selected from the liver disorders (CHC, CHB, NASH) and normal liver (NL), selection of miRNAs that can be discriminated with favorable accuracy was performed. The results are shown in Table 5 (Table 5-1 to Table 5-6) below.

### [Table 5]

**(Table 5)**

| Table 5-1 | Result | | | |
|---|---|---|---|---|
| prediction | | NASH | NL | |
| | NASH | 7 | 4 | |
| | NL | 0 | 16 | accuracy 85.2% |
| | | | | |

| Table 5-2 | result | | | |
|---|---|---|---|---|
| prediction | | CHC | NASH | |
| | CHC | 62 | 1 | |
| | NASH | 1 | 7 | accuracy 97.2% |
| | | | | |

| Table 5-3 | result | | | |
|---|---|---|---|---|
| prediction | | CHB | NASH | |
| | CHB | 2 | 1 | |
| | NASH | 2 | 6 | accuracy 72.7% |
| | | | | |

| Table 5-4 | result | | | |
|---|---|---|---|---|
| prediction | | CHC | NL | |
| | CHC | 64 | 2 | |
| | NL | 0 | 18 | accuracy 97.6% |
| | | | | |

| Table 5-5 | result | | | |
|---|---|---|---|---|
| prediction | | CHB | NL | |
| | CHB | 3 | 5 | |
| | NL | 1 | 15 | accuracy 75.0% |
| | | | | |

| Table 5-6 | result | | | |
|---|---|---|---|---|
| prediction | | CHB | CHC | |
| | CHB | 3 | 0 | |
| | CHC | 1 | 64 | accuracy 98.5% |

Table 6 below shows miRNAs used for discriminating the six pairs in Table 5. The expression levels of miRNAs indicated by "*" in Table 6 were used.

### [Table 6]

**(Table 6)**

| miRNA | NASHvsNL | NASHvsCHC | NASHvsCHB | NLvsCHC | NLvsCHB | CHCvsCHB |
|---|---|---|---|---|---|---|
| hsa-miR-451a | * | * | * | * | * | * |
| hsa-miR-638 | * | * | | * | * | * |
| hsa-miR-762 | * | * | | * | * | * |
| hsa-miR-320c | * | * | | * | * | * |
| hsa-miR-92a | * | * | | * | * | * |
| hsa-miR-486-5p | * | * | | * | * | * |
| hsa-miR-630 | * | * | | * | * | * |
| hsa-miR-1246 | * | * | | * | * | * |
| hsa-miR-1225-5p | * | * | | * | | * |
| hsa-miR-1275 | * | * | | * | | * |
| hsa-miR-720 | * | * | | * | | * |
| hsa-miR-1202 | * | * | | * | | * |
| hsa-miR-1207-5p | * | * | | * | | * |
| hsa-miR-320d | * | * | | * | | * |
| hsa-miR-22 | * | * | | | | * |
| hsa-miR-1915 | * | * | | | | * |
| hsa-miR-483-5p | | * | | * | | * |
| hsa-miR-320b | | * | | | | * |
| hsa-miR-1268 | | * | | | | * |
| hsa-miR-1 | * | | | | | |
| hsa-miR-16 | * | | | | | |

Table 7 below shows the result of a calculation performed by organizing the results in Table 5 into a 4x4 contingency table for calculating the accuracy.

### [Table 7]

**(Table 7)**

| | Result | | | | | |
|---|---|---|---|---|---|---|
| prediction | | CHC | NL | CHB | NASH | |
| | CHC | 62 | 2 | 1 | 2 | |
| | NL | 0 | 16 | 0 | 0 | |
| | CHB | 0 | 1 | 3 | 2 | accuracy 88.40% |
| | NASH | 2 | 1 | 2 | 3 | |

As shown in Tables 5 to 7, it has been found that classification of liver disorders (CHC, CHB, NASH) and normal liver can be performed based on the expression levels of miRNAs in the blood samples shown in Table 5.

### 8. Analytical result for a case of increasing samples from NASH patients

Peripheral blood samples were obtained from twenty NASH patients and twenty-four persons having normal livers so as to perform an analysis on the pairs of NASH and normal liver (NL). The operation was conducted similarly to the above Table 2-1 except that five combinations of hsa-miR-638, hsa-miR-762, hsa-miR-451a, hsa-miR-1974, and hsa-miR-1246 were used for the non-coding RNA. The results are shown in Table 8 below.

### [Table 8]

**(Table 8)**

| | Result | | | |
|---|---|---|---|---|
| prediction | | NASH | NL | |
| | NASH | 18 | 5 | |
| | NL | 2 | 19 | accuracy 84.1% |

Table 8 above shows that NASH and normal liver (NL) can be discriminated from each other with a favorable accuracy.

### Industrial Applicability

The present application is used favorably in the pharmaceutical development regarding liver disorders, the medical field of liver disorders, the research field of liver disorders and the like.

### Free text of sequence listing

Sequence ID Nos. 1-23: Sequence of non-coding RNA (see Table 1)

## Claims

1. A method for classifying test body fluid samples into either of two groups selected from four groups consisting of chronic hepatitis C, chronic hepatitis B, non-alcoholic steatohepatitis (NASH) and normal liver, the method comprising a measurement of an expression level of one or plural non-coding RNA(s) in the test body fluid samples.

2. The method according to claim 1, wherein the one or plural non-coding RNA(s) comprises hsa-miR-451a.

3. The method according to claim 1 or 2, further comprising discriminating to which of the two groups the test body fluid samples belong, by use of the expression level of the one or plural non-coding RNA(s) in the test body fluid samples and sample data information obtained from standard body fluid samples of the two groups.

4. A method for classifying a test body fluid sample as either non-alcoholic steatohepatitis (NASH) or normal liver,
wherein the method comprises a measurement of an expression level of one or plural non-coding RNA(s) in the test body fluid sample, and
the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-1271-5p, hsa-miR-22-3p, hsa-miR-1, hsa-miR-16-5p, and a combination thereof.

5. A method for classifying a test body fluid sample as either non-alcoholic steatohepatitis (NASH) or chronic hepatitis C,
wherein the method comprises a measurement of an expression level of one or plural non-coding RNA(s) in the test body fluid sample, and
the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-483-5p, hsa-miR-320b, hsa-miR-1268a, and a combination thereof.

6. A method for classifying a test body fluid sample as either non-alcoholic steatohepatitis (NASH) or chronic hepatitis B,
wherein the method comprises a measurement of an expression level of one or plural non-coding RNA(s) in the test body fluid sample, and
the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-720, and a combination thereof.

7. A method for classifying a test body fluid sample as either chronic hepatitis C or normal liver,
wherein the method comprises a measurement of an expression level of one or plural non-coding RNA(s) in the test body fluid sample, and
the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-1225-5p, hsa-miR-1275, hsa-miR-1207-5p, and a combination thereof.

8. A method for classifying a test body fluid sample as either chronic hepatitis B or normal liver,
wherein the method comprises a measurement of an expression level of one or plural non-coding RNA(s) in the test body fluid sample, and
the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1271-5p, hsa-miR-22-3p, and a combination thereof.

9. A method for classifying a test body fluid sample as either chronic hepatitis B or chronic hepatitis C,
wherein the method comprises a measurement of an expression level of one or plural non-coding RNA(s) in the test body fluid sample, and
the one or plural non-coding RNA(s) is selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-483-5p, hsa-miR-320b, hsa-miR-1268a, and a combination thereof.

10. The method according to any one of claims 1 to 3, wherein the non-coding RNA is a mircoRNA.

11. A method for classifying a test body fluid sample as either non-alcoholic steatohepatitis (NASH) or normal liver,
wherein the method comprises a measurement of an expression level of one or plural mircoRNA(s) in the test body fluid sample, and
the one or plural mircoRNA(s) is selected from the group consisting of: hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-1, hsa-miR-16, and a combination thereof.

12. A method for classifying a test body fluid sample as either non-alcoholic steatohepatitis (NASH) or chronic hepatitis C,
wherein the method comprises a measurement of an expression level of one or plural mircoRNA(s) in the test body fluid sample, and
the one or plural mircoRNA(s) is selected from the group consisting of: hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-483-5p, hsa-miR-320b, hsa-miR-1268, and a combination thereof.

13. A method for classifying a test body fluid sample as either non-alcoholic steatohepatitis (NASH) or chronic hepatitis B,
wherein the method comprises a measurement of an expression level of a mircoRNA in the test body fluid sample, and
the mircoRNA is hsa-miR-451a.

14. A method for classifying a test body fluid sample as either chronic hepatitis C or normal liver,
wherein the method comprises a measurement of an expression level of one or plural mircoRNA(s) in the test body fluid sample, and
the one or plural mircoRNA(s) is selected from the group consisting of: hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-483-5p, and a combination thereof.

15. A method for classifying a test body fluid sample as either chronic hepatitis B or normal liver,
wherein the method comprises a measurement of an expression level of one or plural mircoRNA(s) in the test body fluid sample, and
the one or plural mircoRNA(s) is selected from the group consisting of: hsa-miR-451a, hsa-miR-638, hsa-miR-762, hsa-miR-320c, hsa-miR-92a, hsa-miR-486-5p, hsa-miR-630, hsa-miR-1246, and a combination thereof.

16. A method for classifying a test body fluid sample as either chronic hepatitis B or chronic hepatitis C,
wherein the method comprises a measurement of an expression level of one or plural mircoRNA(s) in the test body fluid sample, and
the one or plural mircoRNA(s) is selected from the group consisting of: hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-483-5p, hsa-miR-320b, hsa-miR-1268, and a combination thereof.

17. The method according to any one of claims 1 to 16, wherein the body fluid sample is a blood sample.

18. The method according to any one of claims 1 to 17, wherein the non-coding RNA or the mircoRNA is a RNA in an exosome-rich fraction of the body fluid sample.

19. A method for classifying test body fluid samples into any of four groups consisting of chronic hepatitis C, chronic hepatitis B, non-alcoholic steatohepatitis (NASH) and normal liver, the method comprising a classification of the test body fluid samples by using the method according to any one of claims 1 to 18.

20. Use of a non-coding RNA selected from the group consisting of: hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-1271-5p, hsa-miR-22-3p, hsa-miR-483-5p, hsa-miR-320b, hsa-miR-1268a, hsa-miR-1, hsa-miR-16-5p, and a combination thereof, as a marker of chronic hepatitis C, chronic hepatitis B, and/or non-alcoholic steatohepatitis (NASH).

21. Use of a mircoRNA selected from the group consisting of hsa-miR-451a, hsa-miR-638, hsa-miR-762, hsa-miR-320c, hsa-miR-92a, hsa-miR-486-5p, hsa-miR-630, hsa-miR-1246, hsa-miR-1225-5p, hsa-miR-1275, hsa-miR-720, hsa-miR-1202, hsa-miR-1207-5p, hsa-miR-320d, hsa-miR-22, hsa-miR-1915, hsa-miR-483-5p, hsa-miR-320b, hsa-miR-1268, hsa-miR-1, hsa-miR-16, and a combination thereof, as a marker of chronic hepatitis C, chronic hepatitis B, and/or non-alcoholic steatohepatitis (NASH).

22. A quantitative PCR kit used for the method according to any one of claims 1 to 19, comprising a primer and a reagent capable of measuring an expression level of one or plural non-coding RNA(s) in the test body fluid sample.

23. A microarray used for the method according to any one of claims 1 to 19, comprising a probe capable of measuring an expression level of one or plural non-coding RNA(s) in the test body fluid sample.

24. A method of using the kit according to claim 22 or the microarray according to claim 23, comprising a measurement of the one or plural non-coding RNA(s) in the method according to any one of claims 1 to 19, using the kit or the microarray.

25. A data set comprising expression data of one or plural non-coding RNA(s) selected from the group consisting of hsa-miR-638, hsa-miR-762, hsa-miR-320c, hsa-miR-451a, hsa-miR-1974, hsa-miR-1246, hsa-miR-92a-3p, hsa-miR-486-5p, hsa-miR-630, hsa-miR-1225-5p, hsa-miR-1275, hsa-miR-720, hsa-miR-1207-5p, hsa-miR-1202, hsa-miR-1915-3p, hsa-miR-320d, hsa-miR-1271-5p, hsa-miR-22-3p, hsa-miR-483-5p, hsa-miR-320b, hsa-miR-1268a, hsa-miR-1, hsa-miR-16-5p, and a combination thereof in the body fluid sample,
the data set comprises expression data of said non-coding RNA obtained from a standard body fluid sample of an individual belonging to at least one group selected from four groups consisting of chronic hepatitis C, chronic hepatitis B, non-alcoholic steatohepatitis (NASH) and normal liver, and the data set is to be used in the method according to any one of claims 1 to 19.

26. Use of a next-generation sequencer in the method according to any one of claims 1 to 19, comprising a measurement of an expression level of one or plural non-coding RNA(s) in the test body fluid sample by use of the next-generation sequencer.

27. A kit for performing the method according to any one of claims 1 to 19 with a next-generation sequencer, the kit comprising a primer and a reagent capable of measuring an expression level of the one or plural non-coding RNA(s) in the test body fluid sample.


SEQUENCE LISTING

<110>  SUMITOMO BAKELITE CO., LTD.

<120>  Method of Classification of Biological Fluid Test Sample

<130>  H4097

<150>  JP 2012/37586
<151>  2012-02-23

<160>  23

<170>  PatentIn version 3.5

<210>  1
<211>  25
<212>  RNA
<213>  Homo sapiens

<400>  1
agggaucgcg ggcggguggc ggccu                                              25


<210>  2
<211>  22
<212>  RNA
<213>  Homo sapiens

<400>  2
ggggcugggg ccggggccga gc                                                 22


<210>  3
<211>  20
<212>  RNA
<213>  Homo sapiens

<400>  3
aaaagcuggg uugagagggu                                                    20


<210>  4
<211>  22
<212>  RNA
<213>  Homo sapiens

<400>  4
aaaccguuac cauuacugag uu                                                 22


<210>  5
<211>  16
<212>  RNA
<213>  Homo sapiens

<400>  5
aguccgugcg agaaua                                                        16


<210>  6
<211>  19
<212>  RNA

<213>  Homo sapiens

<400>  6
aauggauuuu uggagcagg                                                    19


<210>  7
<211>  22
<212>  RNA
<213>  Homo sapiens

<400>  7
uauugcacuu gucccggccu gu                                                22


<210>  8
<211>  22
<212>  RNA
<213>  Homo sapiens

<400>  8
uccuguacug agcugccccg ag                                                22


<210>  9
<211>  22
<212>  RNA
<213>  Homo sapiens

<400>  9
aguauucugu accagggaag gu                                                22


<210>  10
<211>  22
<212>  RNA
<213>  Homo sapiens

<400>  10
gugguacgg cccaguggggg gg                                                22


<210>  11
<211>  17
<212>  RNA
<213>  Homo sapiens

<400>  11
   gugggggaga ggcuguc                                                       17


<210>  12
<211>  17
<212>  RNA
<213>  Homo sapiens

<400>  12
ucucgcuggg gccucca                                                      17


<210>  13
<211>  21
<212>  RNA

<213>  Homo sapiens

<400>  13
uggcagggag gcugggaggg g                                                21


<210>  14
<211>  21
<212>  RNA
<213>  Homo sapiens

<400>  14
gugccagcug caguggggga g                                                21


<210>  15
<211>  20
<212>  RNA
<213>  Homo sapiens

<400>  15
ccccagggcg acgcggcggg                                                  20


<210>  16
<211>  19
<212>  RNA
<213>  Homo sapiens

<400>  16
aaaagcuggg uugagagga                                                   19


<210>  17
<211>  22
<212>  RNA
<213>  Homo sapiens

<400>  17
cuuggcaccu agcaagcacu ca                                               22


<210>  18
<211>  22
<212>  RNA
<213>  Homo sapiens

<400>  18
aagcugccag uugaagaacu gu                                               22


<210>  19
<211>  22
<212>  RNA
<213>  Homo sapiens

<400>  19
aagacgggag gaaagaaggg ag                                               22


<210>  20
<211>  22
<212>  RNA

<213>  Homo sapiens

<400>  20
aaaagcuggg uugagagggc aa                                                22


<210>  21
<211>  18
<212>  RNA
<213>  Homo sapiens

<400>  21
   cgggcguggu gguggggg                                                      18


<210>  22
<211>  22
<212>  RNA
<213>  Homo sapiens

<400>  22
uggaauguaa agaaguaugu au                                                22


<210>  23
<211>  22
<212>  RNA
<213>  Homo sapiens

<400>  23
uagcagcacg uaaauauugg cg                                                22